# EUROPEAN PATENT SPECIFICATION

(11) **EP 3 461 448 B1**
(45) Date of publication and mention of the grant of the patent: **17.02.2021**
(21) Application number: 18196606.0
(22) Date of filing: 25.09.2018
(51) Int. Cl.: A61B 18/18

(54) **FLEXIBLE ABLATION CATHETER WITH STIFF SECTION AROUND RADIATOR**
FLEXIBLER ABLATIONSKATHETER MIT STEIFEM ABSCHNITT UM DEN STRAHLER HERUM
CATHÉTER D'ABLATION SOUPLE AVEC SECTION RIGIDE AUTOUR DE RADIATEUR

(30) Priority: 26.09.2017 US 201762563317 P; 17.09.2018 US 201816132755
(43) Date of publication of application: 03.04.2019
(73) Proprietor: Covidien LP, Mansfield, MA 02048 (US)
(72) Inventor: DICKHANS, William J., Longmont, CO Colorado 80503 (US)
(74) Representative: Maschio, Antonio

(56) References cited:
- US-A1- 2011 238 055
- US-A1- 2012 259 326
- US-B1- 6 251 128

## Description

### BACKGROUND

### 1. Technical Field

The present disclosure is directed to a water-jacketed microwave ablation antenna assembly and more particularly to a microwave ablation antenna with sufficient rigidity to enable for insertion into desired tissue and to withstand being grasped and sufficient flexibility to permit navigation around organs and other structures.

### 2. Description of Related Art

Microwave ablation antennae are well-known mechanisms for treating cancerous lesions and tumors in the body. For example, treatment of liver tumors is often undertaken by the placement of one or more microwave ablation antennae proximate the tumor and then treatment with microwave radiation up to and exceeding 150W of power for a duration sufficient to coagulate and kill the tissue of the tumor and some margin of healthy tissue.

Some microwave ablation antennae are cooled using compressed or liquefied CO₂ gas, which during expansion absorbs energy from the antenna and particularly the coaxial cabling to help limit damage to healthy tissue proximate the radiating section which is normally formed on a distal portion of the antenna. The actual radiator of such devices is often separated from the cooling gas flow.

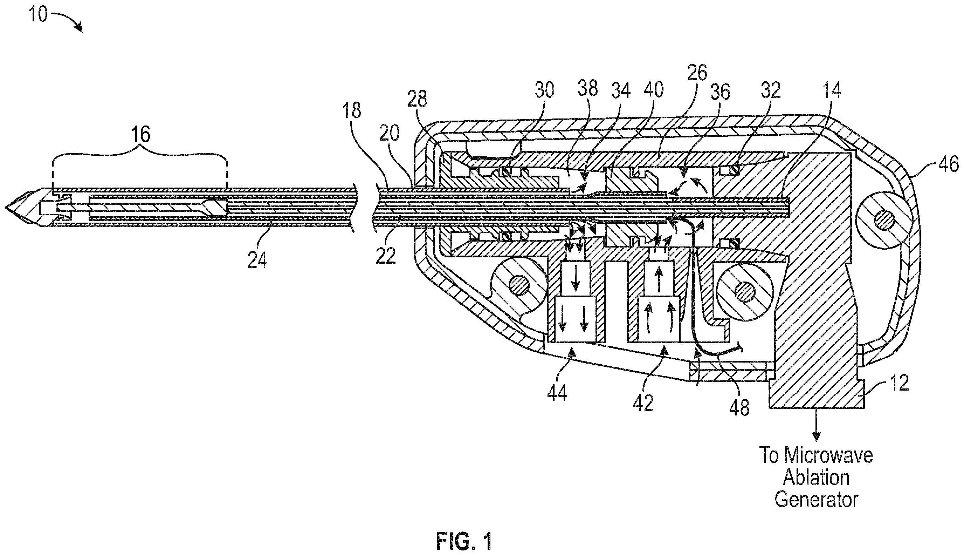

In an alternative arrangement, a circulating fluid, generally saline or deionized water, is pumped through the microwave ablation antenna assembly. One such configuration has been described in detail in commonly assigned U.S. Patent No. 9,119,650, entitled "MICROWAVE ENERGY-DELIVERY DEVICE AND SYSTEM". Fig. 1 depicts a water-jacketed microwave ablation antenna assembly 10 configured for circulating a fluid therethrough. As shown in Fig. 1, the microwave ablation assembly 10 includes a transition 12, which connects via a coaxial cable to a microwave ablation generator (not shown). The transition 12 allows for a 90° change in direction of the coaxial cable entering the transition 12 to the coaxial cable 14 of the microwave ablation assembly 10. The coaxial cable 14 extends perpendicularly from the transition 12 and concludes at a radiating section 16. The radiating section 16 may take many forms including monopole, dipole, symmetric and asymmetric configurations. The coaxial cable 14 extends through a first tubular member 18, which is itself housed within a second tubular member 20. Between the coaxial cable 14 and the first tubular member 18 is a first fluid channel 22 and between the first tubular member 18 and the second tubular member 20 is a second fluid channel 24. The transition 12 is received within a first end of a hub 26, a hub cap 28 is received at a second end of the hub 26, and is itself designed to receive and secure the second tubular member 20. O-rings 30 and 32 formed on the hub cap 28 and the transition 12, form seals creating a watertight compartment 34 there between.

Further, as shown in Fig. 1, the watertight compartment 34 is separated into inflow chamber 36 and outflow chamber 38 by hub divider 40. The hub divider 40 receives the first tubular member 18 and maintains it in alignment with the second tubular member 20. The hub divider 40 is formed of an elastomeric material and forms a seal around the first tubular member 18 which, in combination with a compression fit within the hub 26, restricts the egress of fluid in inflow chamber 36 to the first fluid channel 22, and prevents fluid returning through second fluid channel 24 and entering outflow chamber 38 from re-entering the inflow chamber 36. Also shown in Fig. 1 are inflow port 42 and outflow port 44 which connect to inflow chamber 36 and outflow chamber 38, respectively. A wire 48 is depicted extending through the hub 26 and inflow chamber 36 and entering the first tubular member 18 where it will terminate at a point proximate the radiating section 16 and include a thermocouple (not shown) to detect the temperature or the microwave ablation assembly 10. The entire hub 26, hub cap 28, and transition 12, once assembled, are placed within a handle assembly 46 for ease of gripping and other ergonomic concerns.

The microwave ablation antenna assembly 10 is quite successful commercially and is currently sold by Medtronic as part of the EMPRINT™ ablation system, however, improvements are always desirable.

US 2012259326 discloses a method for forming a resonating structure within a body lumen, the method including advancing a flexible microwave catheter into a body lumen of a patient, the flexible microwave catheter including a radiating portion at the distal end of the flexible microwave catheter, the radiating portion configured to receive microwave energy, and at least one centering device proximate the radiating portion configured to deploy radially outward from the flexible microwave catheter; positioning the radiating portion near tissue of interest; deploying the at least one centering device radially outward from the flexible microwave catheter within the body lumen such that a longitudinal axis of the radiating portion is substantially parallel with and at a fixed distance from a longitudinal axis of the body lumen near the targeted tissue; and delivering microwave energy to the radiating portion such that a circumferentially balanced resonating structure is formed with the body lumen. In this context, reference is also made to document US2011/0238055 A1.

### SUMMARY

One aspect of the present disclosure is directed to a microwave ablation catheter as defined in claim 1. The microwave ablation catheter includes a radiating section formed at a distal end of a coaxial cable, an inner tubular member circumscribing the coaxial cable and at least a portion of the radiating section, and an outer tubular member circumscribing the inner tubular member, the outer tubular member including a flexible portion and a rigid portion. The microwave ablation catheter further includes a tube for injection of one or more agents to promote adhesion of the microwave ablation catheter to surrounding tissue in which it is inserted.

The rigid portion of the outer tubular member may substantially circumscribe the radiating section. Alternatively, the rigid portion of the outer tubular member may be formed proximal of the radiating section. Additionally, the outer tubular member may be flexible both proximal and distal of the rigid portion. The rigid portion may be formed of a rigid sleeve, and the rigid sleeve may be adhered to an exterior surface of the outer tubular member.

The rigid portion of the outer tubular member may be configured for grasping by a laparoscopic grasping tool, and may prevent damage to structures of the microwave ablation catheter.

The microwave ablation catheter may be configured to be received in a laparoscopic port, and may include a water jacket.

The microwave ablation catheter may further include a tissue retention member. The tissue retention member may be a balloon or at least one barb. In embodiments including a barb, the microwave ablation catheter may further include a retractable sleeve, and the barb may be biased away from the outer tubular member. Retraction of the retractable sleeve may release the barb. Alternatively, the at least one barb may be formed of a flexible material on an exterior surface of the outer tubular member.

### BRIEF DESCRIPTION OF THE FIGURES

Objects and features of the present disclosure will become apparent to those of ordinary skill in the art when descriptions of various embodiments thereof are read with reference to the accompanying drawings, of which:
Fig. 1 is a cross-sectional view of a known microwave ablation antenna assembly;
Fig. 2 is a view of a microwave ablation assembly according to the present disclosure in use partially in a patient;
Fig. 3 is a cross-sectional view of a distal portion of a microwave ablation assembly in accordance with the present disclosure; and
Figs. 4A-4D depict a variety of methods for adhering a microwave ablation assembly in position within target tissues.

### DETAILED DESCRIPTION

With respect to the microwave ablation assembly 10 depicted in Fig. 1, because the second tubular member 20 is rigid, the microwave ablation antenna assembly 10 is primarily useful for percutaneous and open surgeries. While use in laparoscopic surgery is possible, the rigidity impedes the ability of the surgeon to properly place the microwave ablation assembly at the target tissue for treatment. Further, fully flexible ablation catheters have been designed and developed for use in lung ablation techniques, for example those described in U.S. Patent Application No. 9,247,992, entitled MICROWAVE ABLATION CATHETER AND METHOD OF UTILIZING THE SAME to Ladtkow, et al.. In place of rigid tubular members for the water-jacketing, these solutions employ flexible catheters. This change makes them excellent at navigating the tortuous pathways of the lungs or other luminal structures where multiple twists and bends are necessary for placement. However, these devices are not an ideal solution for utilization in laparoscopic surgery. The flexibility and the lack of a rigid portion at the distal end of the devices can make it difficult to pierce tissue and place a radiating section within a target. Further, the flexible nature of the catheters and the materials they are made of makes such solutions susceptible to damage when handled by laparoscopic graspers and the like.

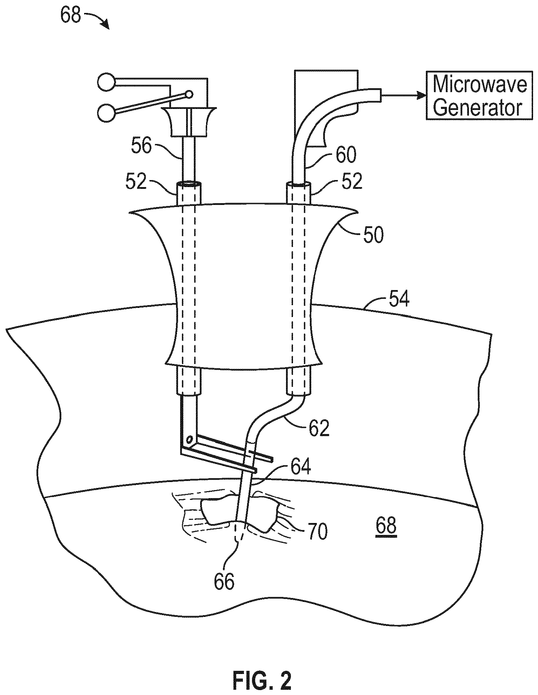

The present disclosure is directed to an ablation probe suitable for placement during laparoscopic and open surgeries. Fig. 2 depicts a laparoscopic port 50 having two laparoscopic channels 52 for receiving instruments. The laparoscopic port 50 has been placed through the tissue of a patient 54 (e.g., through the abdominal wall and related tissues). Those of skill in the art will recognize that a second port 50 may be employed to insufflate the abdomen and provide space for accessing the desired organs, and navigation of the surgical instruments. The second port 50 may also allow for visualization of the surgical site via a laparoscope (not shown) inserted therethrough. In Fig. 2, a laparoscopic grasper 56 is inserted through one of the channels 52.

A microwave ablation probe 60 in accordance with the present disclosure is inserted through a second laparoscopic channel 52. The microwave ablation probe 60 is electrically connected to a microwave ablation generator, which may include a pump for circulating fluid through the microwave ablation probe 60. The microwave ablation probe 60 includes a flexible portion 62 and a rigid portion 64. As depicted in Fig. 2, the rigid portion 64 includes the distal end 66 of the microwave ablation probe 60. Within the rigid portion 64 is a water-jacketed coaxial cable and radiating section described in greater detail below. The flexibility of the flexible portion 62 allows for the microwave ablation probe 60 to be maneuvered within the insufflated abdominal cavity by the laparoscopic grasper 56, for placement in a desired location in an organ 68, for treatment of a target 70. The rigid portion 64, which may include the distal end 66, provides column strength for that portion of the microwave ablation probe 60 enabling it to be inserted into and to pierce tissue of organs such as the liver, kidneys, spleen, lungs, etc. The rigid portion also allows the surgeon to grasp the microwave ablation probe 60 using the laparoscopic graspers 56. Those of skill in the art will recognize that while exemplary graspers are depicted in Fig. 2 the present disclosure is not so limited and the rigid portion 64 of the microwave ablation probe 60 may be grasped, handled, and maneuvered by any commonly utilized laparoscopic tool.

In operation, following insertion through the laparoscopic channel 52, the surgeon utilizes the graspers 56 to grasp the rigid distal portion 64. Then the surgeon can maneuver the rigid distal portion 64 proximate the target 70. The flexible portion 62 permits bending and twisting of the microwave ablation probe 60 as necessary to avoid critical structures, limit undesired damage to neighboring tissues, and in general allow the laparoscopic grasper to properly place the rigid distal portion, 64, in which may be housed the radiating section, as described in greater detail below. Placement of the rigid distal section 64 may be accompanied by visualization via a laparoscope, as well as a variety of imaging techniques. For example, via a separate port 50, a laparoscopic ultrasound wand may be inserted and navigated proximate the target 70. Placement of the rigid distal portion 64 may be under ultrasound visualization to ensure that the rigid distal portion 64 is properly placed and critical structures within the organ 68 are avoided and proper margins are available to successfully treat the target 70. Additionally, or alternatively, CT image guidance may be employed to ensure proper placement.

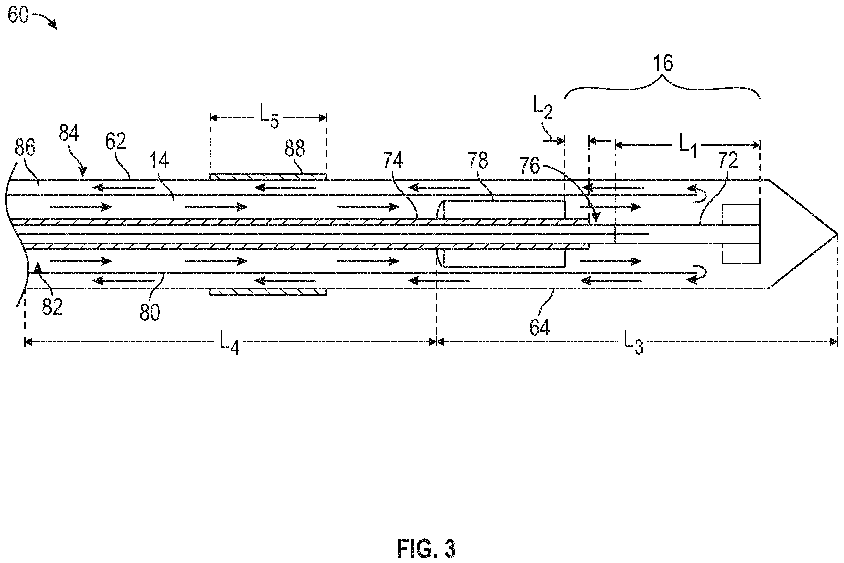

Fig. 3 depicts at least two alternative embodiments of the water-jacketed microwave ablation probe 60. The microwave ablation probe 60 includes a coaxial cable 14 terminating at a radiating section 16. The radiating section 16 includes a distal radiating portion 72 having a length L1 and a proximal radiating portion 74 having a length L2, separated by a feedgap 76. As depicted, the radiating section 16 defines an unbalanced dipole microwave radiator, though a balanced dipole or a monopole radiator are also contemplated within the present disclosure. Proximal of the proximal radiating portion 74 is a balun structure 78. The balun structure may be for example a ¼ wavelength balun that is physically shorted to an outer conductor of the coaxial cable 14, though other balun structures, including un-shorted fluid balun ½ wavelength structures, as well as others, are also within the scope of the present disclosure.

The coaxial cable 14, balun structure, and radiating section 16 are fitted within an inner tubular member 80 and a fluid in-flow channel 82 is formed therebetween. An outer tubular member 84 is formed over the inner tubular member 80 and forms a fluid outflow channel 86 between the inner tubular member 80 and the outer tubular member 84. In this manner, microwave ablation probe 60 has a similar water-jacket to that depicted and described above with respect to Fig. 1. As will be appreciated, fluid in-flow and out-flow may be controlled and directed on a proximal portion of the microwave ablation probe 60 in a similar fashion to that depicted and described with respect to Fig. 1.

In one embodiment of the present disclosure, the outer tubular member 84 of the microwave ablation probe 60 is formed of the flexible proximal portion 62 and the rigid distal portion 64. The flexible proximal portion 62 may be formed of a thermoplastic material, as well as various polyesters and Nylon materials as would be known to those of skill in the art. The rigid distal portion 64 may be formed of a rigid thermoplastic, a fiberglass material, or another nonconductive material. The rigid distal portion 64 may have a length L3 which extends the length of the radiating section 16 and the balun structure 78, thus ensuring that, if grasped by a grasper 56, these portions of the underlying structure are not crushed. The remainder of the length of the microwave ablation probe 60, length L4, may form the flexible proximal portion 62. As will be appreciated, in some embodiments the outer tubular member 84 may include a second rigid portion at a location more proximal than the distal rigid portion 64 along the length of the microwave ablation probe 60. For example, this second rigid portion may largely coincide with the length which remains in the channel 52 of port 50.

In a further embodiment, the distal portion 64 is not rigid, but rather is formed of the same or a similar material as the proximal flexible portion 62, for example, along length L3. Instead, a rigid insert 88 is employed. The rigid insert 88 may be formed of rigid thermoplastic, fiberglass, stainless steel or other appropriate materials to prevent crushing by a grasper 56. The rigid insert 88 may be bonded to the ends of the distal portion 64 and proximal portion 62, or may be a sleeve which is thermo-molded into the material of the outer tubular member 84. Still further, the rigid insert 88 may be a sleeve into which the microwave ablation probe 60 is inserted and subsequently adhered. In some instances this may be located approximately 15 CM from the distal end 66 of the microwave ablation probe 60. This embodiment may be particularly useful where the target 70 is located behind other organs and flexibility of the distal portion 64 of the microwave ablation probe 60 is desirable, but the need to grasp the microwave ablation probe 60 is still necessary. The rigid insert 88 may have a size of approximately 5 CM, though other sizes may be employed as desired. In a further embodiment, the microwave ablation probe 60 may be provided with several sizes of rigid inserts 88 that may be selected as desired by the surgeon for the approach being taken during a procedure. A set-up nurse or other medical professional may then place the rigid insert 88 on the microwave ablation probe 60 and adhere it in the proper location as specified by the surgeon. As will be appreciated, guidance may be provided to the nurse as to the location of the underlying structures to guide in placement.

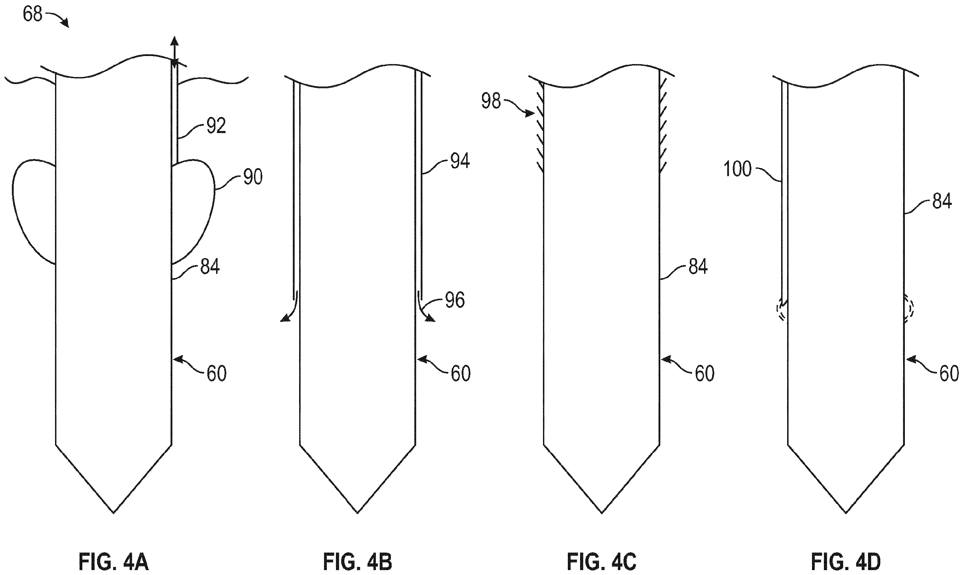

In accordance with a further aspect of the present disclosure, the microwave ablation probe 60 may employ one or more tissue lock mechanisms. Fig. 4A depicts a microwave ablation probe 60 including a balloon 90 formed on the outer tubular member 84. Following insertion into an organ 68, the balloon 90 may be inflated via an inflation tube 92 to hold the microwave ablation probe 60 in position in the organ 68 to be treated. Fig. 4B depicts an alternative arrangement whereby the microwave ablation probe 60 includes a retractable sleeve 94. Upon retraction of the retractable sleeve 94, barbs 96 which are biased away from the microwave ablation probe 60 are uncovered and secure themselves in the tissue of the organ 68 in which the microwave ablation probe 60 has been inserted. To remove the microwave ablation probe 60, the sleeve is forced distally over the barbs 96 to retract them from the tissue of the organ 68 and allow for retraction of the microwave ablation probe 60. Fig. 4C provides a similar barbed solution. However, instead of uncovering them with a retractable sleeve, the outer tubular member 84 has multiple flexible barbs 98 formed thereon. Again, insertion of the microwave ablation probe 60 into tissue of an organ 68 allows the flexible barbs 98 to engage the tissue and limit movement of the microwave ablation probe 60 after release by the grasper 56. However, because of the flexible nature of the flexible barbs 98, application of sufficient force will allow for retraction of the microwave ablation probe 60 without causing significant damage to the tissue of the organ 68. Finally, Fig. 4D depicts a microwave ablation probe 60 having a dispensing tube 100 adhered to an exterior of the outer tubular member 84. The dispensing tube 100 permits the application of coagulants or sealants that may be used to adhere the microwave ablation probe 60 to the tissue of the organ 68. These alternatives may be particularly useful to allow the release of the microwave ablation probe 60 from the grasper 56 following placement and before application of energy.

While several embodiments of the disclosure have been shown in the drawings, it is not intended that the disclosure be limited thereto, as it is intended that the disclosure be as broad in scope as the art will allow and that the specification be read likewise. Any combination of the above embodiments is also envisioned and is within the scope of the appended claims. Therefore, the above description should not be construed as limiting, but merely as exemplifications of particular embodiments. Those skilled in the art will envision other modifications within the scope of the claims appended hereto.

The invention may be described by reference to the following numbered paragraphs:-
1. A microwave ablation catheter comprising:
   a radiating section formed at a distal end of a coaxial cable;
   an inner tubular member circumscribing the coaxial cable and at least a portion of the radiating section; and
   an outer tubular member circumscribing the inner tubular member, the outer tubular member including a flexible portion and a rigid portion.
2. The microwave ablation catheter of paragraph 1, wherein the rigid portion of the outer tubular member substantially circumscribes the radiating section.
3. The microwave ablation catheter of paragraph 1, wherein the rigid portion of the outer tubular member is formed proximal of the radiating section.
4. The microwave ablation catheter of paragraph 3, wherein the outer tubular member is flexible both proximal and distal of the rigid portion.
5. The microwave ablation catheter of paragraph 4, wherein the rigid portion is formed of a rigid sleeve.
6. The microwave ablation catheter of paragraph 5, wherein the rigid sleeve is adhered to an exterior surface of the outer tubular member.
7. The microwave ablation catheter of paragraph 1, wherein the rigid portion is configured for grasping by a laparoscopic grasping tool.
8. The microwave ablation catheter of paragraph 7, wherein the rigid portion prevents damage to structures of the microwave ablation catheter.
9. The microwave ablation catheter of paragraph 1, wherein the outer tubular member is configured to be received in a laparoscopic port.
10. The microwave ablation catheter of paragraph 1, further comprising a water jacket.
11. The microwave ablation catheter of paragraph 1, further comprising a tissue retention member.
12. The microwave ablation catheter of paragraph 11, wherein the tissue retention member is a balloon.
13. The microwave ablation catheter of paragraph 11, wherein the tissue retention member is at least one barb.
14. The microwave ablation catheter of paragraph 13, further comprising a retractable sleeve, wherein the at least one barb is biased away from the outer tubular member and retraction of the retractable sleeve releases the barb.
15. The microwave ablation catheter of paragraph 13, wherein the at least one barb is formed of a flexible material on an exterior surface of the outer tubular member.
16. The microwave ablation catheter of paragraph 11, further comprising a tube for injection of one or more agents to promote adhesion of the microwave ablation catheter to surrounding tissue in which it is inserted.

## Claims

1. A microwave ablation catheter comprising:
a radiating section (16) formed at a distal end of a coaxial cable (14);
an inner tubular member (18) circumscribing the coaxial cable and at least a portion of the radiating section;
an outer tubular member (20) circumscribing the inner tubular member, the outer tubular member including a flexible portion (62) and a rigid portion (64);
and **characterised by** a tube (100) for injection of one or more agents to promote adhesion of the microwave ablation catheter to surrounding tissue in which it is inserted.

2. The microwave ablation catheter of claim 1, wherein the rigid portion of the outer tubular member substantially circumscribes the radiating section.

3. The microwave ablation catheter of claim 1 or claim 2, wherein the rigid portion of the outer tubular member is formed proximal of the radiating section.

4. The microwave ablation catheter of claim 3, wherein the outer tubular member is flexible both proximal and distal of the rigid portion.

5. The microwave ablation catheter of claim 4, wherein the rigid portion is formed of a rigid sleeve.

6. The microwave ablation catheter of claim 5, wherein the rigid sleeve is adhered to an exterior surface of the outer tubular member.

7. The microwave ablation catheter of any preceding claim, further comprising a water jacket.

8. The microwave ablation catheter of any preceding claim, further comprising a tissue retention member.

9. The microwave ablation catheter of claim 8, wherein the tissue retention member is a balloon (90).

10. The microwave ablation catheter of claim 8, wherein the tissue retention member is at least one barb (96).

11. The microwave ablation catheter of claim 10, further comprising a retractable sleeve (94), wherein the at least one barb is biased away from the outer tubular member and retraction of the retractable sleeve releases the barb.

12. The microwave ablation catheter of claim 11, wherein the at least one barb is formed of a flexible material on an exterior surface of the outer tubular member.

13. Apparatus including the microwave ablation catheter of any preceding claim and a laparoscopic grasping tool, wherein the rigid portion is configured for grasping by the laparoscopic grasping tool.

14. The apparatus of claim 13, wherein the rigid portion is configured to prevent damage to structures of the microwave ablation catheter when handled by the laparoscopic grasping tool.

15. The apparatus of claim 13 or 14, further comprising a laparoscopic port (50), and wherein the outer tubular member is configured to be received in the laparoscopic port.

## Patentansprüche

1. Mikrowellenablationskatheter, umfassend:
einen Strahlungsteil (16), der an einem distalen Ende eines Koaxialkabels (14) ausgebildet ist;
ein inneres rohrförmiges Element (18), das das Koaxialkabel und mindestens einen Abschnitt des Strahlungsteils umschreibt;
ein äußeres rohrförmiges Element (20), das das innere rohrförmige Element umschreibt, wobei das äußere rohrförmige Element einen flexiblen Abschnitt (62) und einen starren Abschnitt (64) enthält; und
**gekennzeichnet durch** einen Schlauch (100) zur Injektion eines oder mehrerer Mittel zur Förderung der Haftung des Mikrowellenablationskatheters an dem umgebenden Gewebe, in das er eingeführt wird.

2. Mikrowellenablationskatheter nach Anspruch 1, wobei der starre Abschnitt des äußeren rohrförmigen Elements den Strahlungsteil im Wesentlichen umschreibt.

3. Mikrowellenablationskatheter nach Anspruch 1 oder 2, wobei der starre Abschnitt des äußeren rohrförmigen Elements proximal des Strahlungsteils ausgebildet ist.

4. Mikrowellenablationskatheter nach Anspruch 3, wobei das äußere rohrförmige Element sowohl proximal als auch distal des starren Abschnitts flexibel ist.

5. Mikrowellenablationskatheter nach Anspruch 4, wobei der starre Abschnitt aus einer starren Hülse ausgebildet ist.

6. Mikrowellenablationskatheter nach Anspruch 5, wobei die starre Hülse an einer Außenfläche des äußeren rohrförmigen Elements haftet.

7. Mikrowellenablationskatheter nach einem der vorhergehenden Ansprüche, ferner umfassend einen Wassermantel.

8. Mikrowellenablationskatheter nach einem der vorhergehenden Ansprüche, ferner umfassend ein Geweberetentionselement.

9. Mikrowellenablationskatheter nach Anspruch 8, wobei das Geweberetentionselement ein Ballon (90) ist.

10. Mikrowellenablationskatheter nach Anspruch 8, wobei das Geweberetentionselement mindestens ein Widerhaken (96) ist.

11. Mikrowellenablationskatheter nach Anspruch 10, ferner umfassend eine einziehbare Hülse (94), wobei der mindestens eine Widerhaken von dem äußeren rohrförmigen Element weg vorgespannt ist und das Einziehen der einziehbaren Hülse den Widerhaken freigibt.

12. Mikrowellenablationskatheter nach Anspruch 11, wobei der mindestens eine Widerhaken aus einem flexiblen Material auf einer Außenfläche des äußeren rohrförmigen Elements ausgebildet ist.

13. Vorrichtung, die den Mikrowellenablationskatheter nach einem der vorhergehenden Ansprüche und ein laparoskopisches Greifwerkzeug umfasst, wobei der starre Abschnitt zum Greifen durch das laparoskopische Greifwerkzeug konfiguriert ist.

14. Vorrichtung nach Anspruch 13, wobei der starre Abschnitt I konfiguriert ist, um eine Beschädigung der Strukturen des Mikrowellenablationskatheters zu verhindern, wenn er mit dem laparoskopischen Greifwerkzeug gehandhabt wird.

15. Vorrichtung nach Anspruch 13 oder 14, ferner umfassend einen laparoskopischen Anschluss (50) und wobei das äußere rohrförmige Element konfiguriert ist, um im laparoskopischen Anschluss aufgenommen zu werden.

## Revendications

1. Cathéter d'ablation par micro-ondes comprenant :
une section rayonnante (16) formée à une extrémité distale d'un câble coaxial (14) ;
un élément tubulaire interne (18) entourant le câble coaxial et au moins une partie de la section rayonnante ;
un élément tubulaire externe (20) entourant l'élément tubulaire interne, l'élément tubulaire externe comportant une partie flexible (62) et une partie rigide (64) ; et
**caractérisé par** un tube (100) servant à l'injection d'un ou de plusieurs agents pour favoriser l'adhérence du cathéter d'ablation par micro-ondes au tissu environnant dans lequel il est inséré.

2. Cathéter d'ablation par micro-ondes selon la revendication 1, dans lequel la partie rigide de l'élément tubulaire externe entoure sensiblement la section rayonnante.

3. Cathéter d'ablation par micro-ondes selon la revendication 1 ou 2, dans lequel la partie rigide de l'élément tubulaire externe est formée de manière proximale par rapport à la section rayonnante.

4. Cathéter d'ablation par micro-ondes selon la revendication 3, dans lequel l'élément tubulaire externe est flexible à la fois de manière proximale et distale par rapport à la partie rigide.

5. Cathéter d'ablation par micro-ondes selon la revendication 4, dans lequel la partie rigide est formée d'un manchon rigide.

6. Cathéter d'ablation par micro-ondes selon la revendication 5, dans lequel le manchon rigide adhère à une surface extérieure de l'élément tubulaire externe.

7. Cathéter d'ablation par micro-ondes selon une quelconque revendication précédente, comprenant en outre une chemise d'eau.

8. Cathéter d'ablation par micro-ondes selon une quelconque revendication précédente, comprenant en outre un élément de rétention de tissu.

9. Cathéter d'ablation par micro-ondes selon la revendication 8, dans lequel l'élément de rétention de tissu est un ballonnet (90).

10. Cathéter d'ablation par micro-ondes selon la revendication 8, dans lequel l'élément de rétention de tissu est au moins un ardillon (96).

11. Cathéter d'ablation par micro-ondes selon la revendication 10, comprenant en outre un manchon rétractable (94), l'au moins un barbillon étant sollicité pour s'écarter de l'élément tubulaire externe, et la rétraction du manchon rétractable libérant l'ardillon.

12. Cathéter d'ablation par micro-ondes selon la revendication 11, dans lequel l'au moins un ardillon est formé d'un matériau flexible sur une surface extérieure de l'élément tubulaire externe.

13. Appareil comportant le cathéter d'ablation par micro-ondes selon une quelconque revendication précédente et un outil de préhension laparoscopique, la partie rigide étant conçue pour être saisie par l'outil de préhension laparoscopique.

14. Appareil selon la revendication 13, dans lequel la partie rigide est conçue pour empêcher l'endommagement des structures du cathéter d'ablation par micro-ondes lorsqu'elle est manipulée par l'outil de préhension laparoscopique.

15. Appareil selon la revendication 13 ou 14, comprenant en outre un orifice laparoscopique (50), et l'élément tubulaire externe étant conçu pour être reçu dans l'orifice laparoscopique.
